# EUROPÄISCHE PATENTANMELDUNG

(11) **EP 0 679 719 A2**
(43) Veröffentlichungstag der Anmeldung: **02.11.1995**
(21) Anmeldenummer: 95105342.0
(22) Anmeldetag: 08.04.1995
(51) Int. Cl.: C12P 5/02, B09B 3/00

(54) **Verfahren zur biologischen Behandlung von organische Stoffe enthaltenden Abfällen**

(30) Priorität: 26.04.1994 DE 4414459
(71) Anmelder: DYCKERHOFF & WIDMANN AG, D-81902 München (DE)
(72) Erfinder:
(74) Vertreter: Patentanwälte Möll und Bitterich

(57) **Zusammenfassung**

Bei der biologischen Behandlung von organische Stoffe enthaltenden Abfällen, wie Hausmüll oder dergleichen, werden die Abfälle nach Zerkleinerung und Konditionierung zu einer rühr- und pumpfähigen Suspension und gegebenenfalls nach Hydrolyse und Versäuerung einer Inertisierung unter anaeroben Bedingungen unterzogen. Die Inertisierung erfolgt in kontinuierlichem Durchlauf in mindestens zwei in Reihe hintereinander geschalteten Reaktionsräumen und wird so geführt, daß die Suspension zumindest bei Einspeisung in die einzelnen Reaktionsräume einen zumindest annähernd konstanten Feststoffgehalt aufweist. Zur Erzielung eines konstanten Feststoffgehaltes kann der Suspension in Abhängigkeit von der fortschreitenden Umsetzung der organischen Stoffe in bzw. zwischen den Reaktionsräumen Überschußwasser entzogen bzw. können Feststoffe zugegeben werden. Durch die Reihenschaltung von zwei oder mehr Reaktionsräumen hintereinander gelingt es bei kontinuierlichem Betrieb, für den Abbau schwerer abbaubarer organischer Substanzen eine größere Verweildauer zur Verfügung zu stellen, vor allem aber, die Gefahr des Austrags von jungem, nicht oder nicht vollständig abgebautem Material zu verhindern.

## Beschreibung

Die Erfindung betrifft ein Verfahren zur biologischen Behandlung von organische Stoffe enthaltenden Abfällen, wie Hausmüll oder dergleichen, in einem mehrstufigen Abbauprozeß gemäß dem Oberbegriff des Patentanspruchs 1.

Trotz aller Bemühungen zur Vermeidung und Verwertung von Abfällen, insbesondere Hausmüll, bleibt eine gewisse Menge von Restmüll, der anderweitig entsorgt, d.h. behandelt und abgelagert werden muß. Ziel der Behandlung muß es deshalb vor allem sein, die Deponienachsorge zu minimieren und so Altlasten von morgen zu vermeiden. Ein wichtiges Kriterium ist dabei der Abbaugrad der organischen Substanzen. Je weniger der abzulagernde Abfall noch biologisch aktiv werden kann, d.h. je inerter er ist, desto weniger aufwendig wird die Deponienachsorge sein.

Eine gängige Art der Behandlung von Restmüll ist die Verbrennung; die Errichtung und der Betrieb von Müllverbrennungsanlagen stößt jedoch in weiten Gruppen der Gesellschaft auf erhebliche Schwierigkeiten. Besonderes Gewicht kommt daher der mechanisch-biologischen Behandlung zu.

Die Technik der geordneten Deponie von Hausmüll und hausmüllähnlichen Abfällen führt dazu, daß sich im Inneren der Deponie ein anaerobes Milieu ausbildet. Dadurch kann sich eine hochspezialisierte, symbiotische Biozönose entwickeln, welche die organischen Mullbestandteile zu einem gasförmigen Endprodukt abbaut, das als Deponiegas (Biogas, Faulgas) bezeichnet wird. Der anaerobe Abbau von organischen Materialien bis hin zu Methan und Kohlendioxid erfolgt durch das sequentielle Zusammenwirken von verschiedenen Bakteriengruppen in im wesentlichen vier Abbauschritten. Im ersten Schritt zerlegen die Mikroorganismen die polymeren organischen Substanzen durch Hydrolyse in ihre Bausteine, die im zweiten Schritt von acidogenen Bakterien zu organischen Säuren und Alkoholen abgebaut werden. Im dritten Schritt werden diese Verbindungen von acetogenen Bakterien benutzt und zu Essigsäure, Wasserstoff und Kohlendioxid umgesetzt. Im letzten Schritt schließlich werden Essigsäure, Wasserstoff und Kohlendioxid von methanogenen Bakterien zu hauptsächlich Methan und Kohlendioxid abgebaut.

Diese Vorgänge, die in einer geordneten Deponie relativ langsam über einen Zeitraum von mehreren Jahrzehnten ablaufen, werden in technischem Maßstab für die Gewinnung von Methangas genutzt. In diesem Zusammenhang ist es bekannt, vergärbare Abfälle nach einer mechanischen Vorsortierung zunächst einem Hydrolysereaktor zuzuführen (WO 92/13084). Im Anschluß an die Hydrolyse erfolgt eine Fest-Flüssig-Trennung, wobei die Flüssigphase direkt einer Methanisierung unterzogen und die Feststoffphase entweder zum weiteren Aufschluß organischer Substanz wieder in den ursprünglichen Hydrolysereaktor zurückgeführt oder in eine separate Hydrolysestufe zum weiteren Aufschluß gebracht wird. Der Austrag aus dieser Stufe wird erneut einer Fest-Flüssig-Trennung unterzogen. Während die Flüssigphase wiederum dem Methanreaktor zugeführt wird, werden die nicht vergärbaren Feststoffe entwässert und verbleiben als Hydrolyse- bzw. Gärreststoffe. Das Verfahren arbeitet in kontinuierlicher Betriebsweise.

Wenn auch die anaeroben Umsetzungsvorgänge in derartigen Reaktoren bei technischen Verfahren wesentlich rascher ablaufen als auf einer Deponie, so muß doch ein wirtschaftlicher Weg zwischen der Behältergröße, der Verweildauer der Suspension im Methanreaktor und dem Grad der Mineralisierung der Gärreststoffe gefunden werden. Insbesondere bei einem kontinuierlichen Betrieb eines einzigen Methanreaktors, dem laufend junges, noch nicht vergorenes Material zugeführt wird, ist nicht zu vermeiden, daß auf der Austragsseite auch immer ein gewisser Prozentsatz an jungem, unvergorenem Material abgeführt wird, das den Grad der Mineralisierung der Gärreststoffe beeinträchtigt.

Der Grad der Mineralisierung der organischen Substanz wird limitiert durch die Mineralisierung der schwer abbaubaren Verbindungen. Zu den mengenmäßig bedeutenden Verbindungen der pflanzlichen organischen Substanz zählen Cellulose und Hemicellulose, gefolgt von Lignin. Dabei stellt Lignin eine Schutzsubstanz gegenüber dem mikrobiellen Angriff der Cellulose dar. Die Abbaubarkeit der Cellulose hängt infolge dessen entscheidend davon ab, inwieweit sie mit Lignin inkrustiert, d.h. quasi imprägniert ist.

Vor diesem Hintergrund liegt der Erfindung die Aufgabe zugrunde, mit wirtschaftlichen Mitteln und möglichst rasch eine möglichst vollständige Mineralisierung der Gärreststoffe zu erreichen, um diese umweltverträglich deponieren zu können.

Erfindungsgemäß wird diese Aufgabe durch die im kennzeichnenden Teil des Patentanspruchs 1 angegebenen Merkmale gelöst.

Vorteilhafte Weiterbildungen der Erfindung ergeben sich aus den Unteransprüchen.

Gegenüber dem Stand der Technik besteht der Grundgedanke der Erfindung darin, den Prozeß der Inertisierung des zu einer Suspension aufbereiteten Restmülls in mehreren als Methanreaktoren arbeitenden Behältern in jeweils gleicher Weise hintereinander bei jeweils konstant gehaltenem Feststoffgehalt als Ausgleich für die abgebaute und als Biogas abgeführte organische Substanz ablaufen zu lassen.

Das dabei entstehende Methangas wird energetisch verwertet. Durch die Reihenschaltung von zwei oder mehr Behältern hintereinander, gelingt es einerseits für den Abbau schwerer abbaubarer organischer Substanzen eine entsprechend größere Verweildauer zur Verfügung zu stellen, vor allem aber die Gefahr des Austrags von jungem, noch nicht abgebautem Material weitestgehend zu verhindern. Statistisch geht die Aufenthaltsdauer der Suspension in den einzelnen Behältern als reziproker Multiplikator ein. Durch die Regulierung des Feststoffgehalts wird die Suspension stabil gehalten, d.h. sie verliert die Tendenz zur Separierung, sei es durch Aufschwimmen oder Absinken.

Ein weiterer Ansatzpunkt der Erfindung besteht darin, die organischen Stoffe, die selbst einer anaeroben Mineraliierung nicht zugänglich sind, insbesondere Lignin, und durch ihre Widerstandsfähigkeit andere organische Verbindungen, wie Cellulose, vor einem mirobiellen Angriff schützen, unter selektiven aeroben Milieubedingungen durch spezielle Mikroorganismen derart angreifen zu lassen, daß sie ihre Schutzfunktion verlieren, damit die dann ungeschützten Verbindungen im anaeroben Prozeß mineralisiert werden können. Dieser aerobe Aufschluß organischer Verbindungen kann vor der Hydrolyse der Suspension erfolgen, zweckmäßig durch Pilze, insbesondere Weißfäulepilze. Dabei wird der Prozeß durch Einstellung des pH-Wertes der Suspension so geführt, daß dabei die leicht mineralisierbaren organischen Verbindungen im aeroben Prozeß nicht schon verbraucht werden, da sie dann später bei der Methangewinnung fehlen würden. Der aerobe Aufschluß kann aber auch einem, vorzugsweise dem zweiten Methanreaktor parallel geschaltet erfolgen.

Das Ziel einer raschen und möglichst weitgehenden Mineralisierung der organischen Substanz kann über den Angriff auf Lignin und die dadurch bewirkte Freilegung der Cellulose erreicht werden.

Die Erfindung wird nachstehend anhand eines als Zeichnung beigegebenen Fließschemas näher erläutert.

Nach der Abfallanlieferung 1 werden in einer Fremdstoffabscheidung 2 Fremd- und Störstoffe aussortiert, z.B. mittels Magnetabscheider, Windsichter, Fliehkraftabscheider oder dergleichen. Das Ziel dieses Verfahrensschrittes ist die Abtrennung von Metallen und Mineralstoffen, damit nachfolgende Aggregate, wie z.B. Pumpen und Mühlen, keinem unnötigen Verschleiß unterliegen und damit sowohl die Schadstoffbelastung des weiteren Abfallstromes, als auch das Volumen der Reaktionsbehälter gering gehalten werden können. Diese Vorbehandlung führt zu einer relativen Anreicherung der organischen Substanz, die zum größten Teil durch Mikroorganismen zu Methan und Kohlendioxid abgebaut werden kann.

In einer anschließenden Zerkleinerungsstufe 3 werden die Abfälle zerkleinert, so daß in einem nachgeschalteten Konditionierer 4 unter Zugabe von Wasser eine rühr- und pumpfähige Suspension mit einem Feststoffgehalt von etwa 10 bis 30 %, vorzugsweise 15 bis 20 %, entsteht. Die Zerkleinerung bewirkt vor allem eine Vergrößerung der Oberfläche, wodurch die organischen Feststoffe dem anaeroben Abbau besser zugänglich werden.

Bei der Konditionierung kann über eine Leitung 5 gegebenenfalls auch organisches Material anderer Herkunft, wie z.B. Klärschlamm, Melasse oder dergleichen, zugegeben werden. Durch einen solchen zusätzlichen Eintrag von organischer Substanz können Schwankungen in der Abfallzusammensetzung ausgeglichen werden, so daß der nachfolgende biologische Abbauprozeß stabiler und gleichmäßiger abläuft. Die so vorbehandelte Suspension wird dann über eine Leitung 6, 6a in einen Behälter 7 gepumpt, in dem die organischen Feststoffe durch Hydrolyse und Versäuerung den acetogenen und methanogenen Bakterien zugänglich gemacht werden.

Alternativ zur direkten Überführung in den Hydrolysereaktor 7 kann die mechanisch vorbehandelte und konditionierte Suspension über eine Leitung 6b in einen Behälter 18 gepumpt werden, in dem eine selektive aerobe Behandlung der schwer abbaubaren, andere organische Substanzen imprägnierende Verbindungen durch spezielle Mikroorganismen erfolgt.

Die selektiven Milieubedingungen dieser Behandlungsstufe werden dadurch hergestellt, daß der pH-Wert der Suspension in einen Bereich erniedrigt wird, in dem die einzusetzenden speziellen Mikroorganismen das Optimum ihrer Enzymaktivität aufweisen und gleichzeitig die Mineralisierung der leicht angreifbaren organischen Substanz durch Bakterien weitgehend eingeschränkt ist. Dies wird durch Erniedrigung des pH-Wertes, vorteilhafterweise auf einen Bereich pH = 4,2 bis 4,6, erreicht. Die Erniedrigung des pH-Wertes kann einmal durch Zudosierung eines organischen Puffers über eine Leitung 20 zur vorbehandelten Suspension erfolgen; als organische Puffer eignen sich z.B. Succinat und Phthalat. Alternativ kann der pH-Wert auch über die Einleitung von Kohlendioxid in die Suspension erniedrigt werden.

Da es sich beim Ligninangriff durch die speziellen Mikroorganismen um einen zumindest in der Anfangsphase oxidativen Prozeß handelt, ist es zweckmäßig, über eine Leitung 19 ein sauerstoffhaltiges Gas in die Suspension einzutragen; dies sollte so erfolgen, daß damit zugleich eine Durchmischung der Suspension erfolgt. Als auf den Ligninangriff spezialisierte Mikroorganismen werden zweckmäßig Pilze, insbesondere Weißfäulepilze, eingesetzt, die über eine Leitung 21 zudosiert werden. Da es sich beim Ligninangriff um einen Cometabolismus mit Kohlehydraten handelt, werden vorteilhafterweise solche Stämme eingesetzt, die einen hohen Quotienten zwischen ligninolytischer und cellulolytischer Aktivität aufweisen.

Ein weiterer Vorteil der Erfindung besteht darin, daß die auf den Ligninangriff spezialisierten Pilze, insbesondere die Weißfäulepilze, ein extrazelluläres Enzymsystem bilden, das über einen Radikalmechanismus auch im Restmüll enthaltene, schwer abbaubare Umweltgifte (Xenobiotika, wie chlorierte Aromaten etc.) anzugreifen vermag.

Nach Abschluß dieser vorgeschalteten selektiven aeroben Behandlung wird die Suspension dann unter Anhebung des pH-Wertes in den für die am nachfolgenden anaeroben Prozeß beteiligten Mikroorganismen optimalen Bereich über die Leitung 6c in den Hydrolysereaktor 7 gepumpt.

Nach Durchlaufen der Hydrolysephase wird die Suspension dann in die Methanreaktoren 8 geleitet, in denen der organisch gebundene Kohlenstoff in Biogas umgewandelt wird. Im dargestellten Ausführungsbeispiel sind drei Methanreaktoren 8a, 8b und 8c hintereinander angeordnet, die von der Suspension in kontinuierlichem Durchlauf nacheinander durchlaufen werden.

Dabei erfolgt im ersten Methanreaktor 8a bei einer Verweildauer von etwa 10 bis 20 Tagen zunächst die Umsetzung der leicht abbaubaren Stoffe zu Biogas, das über eine Leitung 9a in eine Sammelleitung 9 zur energetischen Verwertung 12 abgezogen wird. Um den Volumenverlust durch die in diesem Reaktor 8a umgesetzten Feststoffe auszugleichen, wird der über die Leitung 10a abgezogenen Suspension in einer Fest-Flüssig-Trennung 11b überschußwasser so weit entzogen, daß die dem zweiten Reaktor 8b zugeführte Suspension den gleichen Feststoffgehalt aufweist.

In dem zweiten Reaktor 8b, der mit einer Verweildauer von ca. 15 bis 30 Tagen der Umsetzung der schwerer abbaubaren Stoffe dient, wird wiederum über eine Leitung 9b das Biogas abgezogen. Die über eine Leitung 10b abgezogene Suspension wird wiederum in einer Fest-Flüssig-Trennung 11c entwässert, um dem dritten Reaktor 8c wiederum mit gleichem Feststoffgehalt zugeführt zu werden. Um nicht nur zwischen den einzelnen Methanreaktoren 8a, 8b und 8c, sondern auch innerhalb dieser den Feststoffgehalt der Suspension konstant zu halten, kann der Inhalt eines jeden Reaktors 8a, 8b, 8c über Leitungen 22a, 22b und 22c umgewälzt und dabei in den Anlagen zur Fest-Flüssig-Trennung 11a, 11b, 11c entwässert werden, so daß in der gesamten anaeroben Abbaustufe die zu behandelnde Suspension immer einen konstanten Feststoffgehalt aufweist.

Im dritten Reaktor 8c werden bei einer Verweildauer von bis zu 50 Tagen die letzten in der Suspension noch enthaltenen abbaubaren Stoffe umgesetzt und über eine Leitung 9c das Biogas sowie über eine Leitung 10c die Suspension abgezogen, bis in einer letzten Entwässerungsstufe 13 die restlichen Feststoffe von der Flüssigkeit getrennt werden. Die stabilisierten, biologisch inerten Reststoffe können dann bei 14 abgezogen und deponiert werden bzw. noch enthaltene Wertstoffe abgeschieden und aufbereitet werden.

Zur Unterstützung des anaeroben Abbauprozesses kann auch eine aerobe Behandlung durch Bakterien erfolgen. Diese sollte nach dem ersten anaeroben Abbauschritt stattfinden, z.B. in einem Aerob-Reaktor 23, der dem zweiten Methanreaktor 8b parallel geschaltet ist. In diesem Reaktor 23 setzen aerobe Mikroorganismen unter Zufuhr von Sauerstoff, z.B. über die Leitung 19, noch enthaltene organische Feststoffe zu Biomasse um, die wiederum bei Überleitung in den Methanreaktor 8b unter anaeroben Bedingungen zu Biogas umgesetzt werden kann.

Das Überschußwasser aus der Entwässerungsstufe 13 wird über eine Leitung 15 gesammelt und als aufbereitetes Prozeßwasser dem Konditionierer 4 zugeführt oder über eine Leitung 16 zu einer Kläranlage 17 abgeleitet. Das aus den Entwässerungsaggregaten 11a, 11b und 11c über die Leitungen 24a, 24b und 24c abgezogene organisch belastete Suspensionswasser wird über eine Sammelleitung 24 einem weiteren Methanreaktor 25 zugeführt, in dem die darin noch enthaltenen gelösten organischen Anteile zu Biogas umgesetzt werden. Das Biogas wird über eine Leitung 9d der Sammelleitung 9 zur energetischen Verwertung 12 zugeführt. Das durch den biologischen Abbauprozeß aufbereitete Wasser wird über eine Leitung 15a der Prozeßwasserleitung 15 und somit wieder der Weiterverwendung im Konditionierer 4 zugeführt.

Das anfallende Biogas, vorwiegend Methan, wird in Gasmotoren energetisch verwertet, wobei die Abwärme zur Beheizung der Hydrolyse- und Methanreaktoren verwendet werden kann. Elektrische Überschußenergie wird in das öffentliche Netz eingespeist.

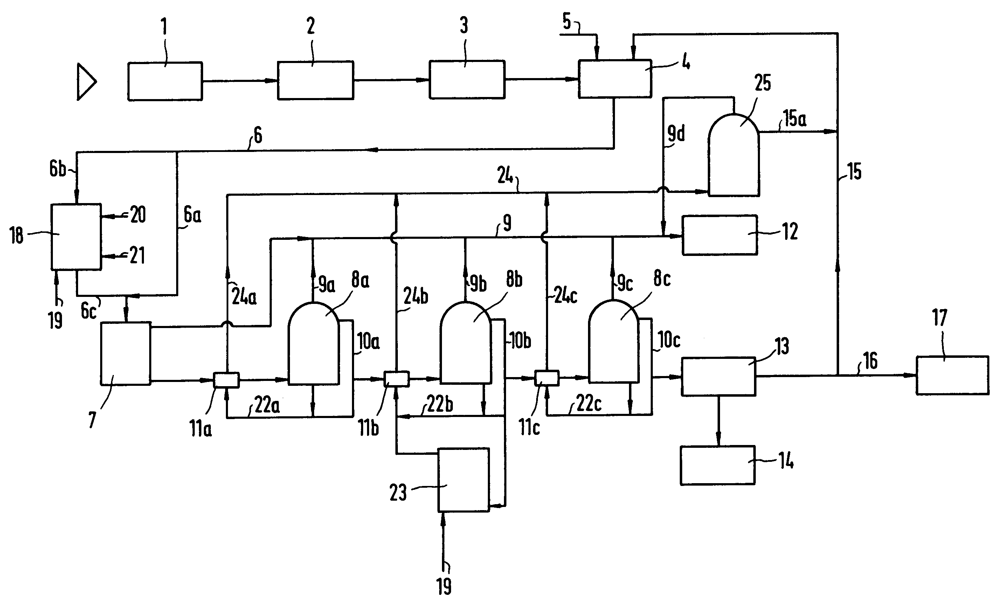

## Patentansprüche

1. Verfahren zur biologischen Behandlung von organische Stoffe enthaltenden Abfällen, wie Hausmüll oder dergleichen, in einem mehrstufigen Abbauprozeß, wobei die Abfälle nach Zerkleinerung und Konditionierung zu einer rühr- und pumpfähigen Suspension einer Inertisierung unter anaeroben Bedingungen unterzogen werden, dadurch gekennzeichnet, daß die Inertisierung in kontinuierlichem Durchlauf in mindestens zwei in Reihe hintereinander geschalteten Reaktionsräumen erfolgt und so geführt wird, daß die Suspension zumindest bei Einspeisung in die einzelnen Reaktionsräume einen zumindest annähernd konstanten Feststoffgehalt aufweist.

2. Verfahren nach Anspruch 1, dadurch gekennzeichnet, daß der Suspension in Abhängigkeit von der fortschreitenden Umsetzung der organischen Stoffe in bzw. zwischen den Reaktionsräumen Überschußwasser entzogen wird.

3. Verfahren nach Anspruch 1, dadurch gekennzeichnet, daß der Suspension in Abhängigkeit von der fortschreitenden Umsetzung der organischen Stoffe in bzw. zwischen den Reaktionsräumen Feststoffe zugegeben werden.

4. Verfahren nach einem der Ansprüche 1 bis 3, dadurch gekennzeichnet, daß der Feststoffgehalt der Suspension in den Reaktionsräumen etwa 10 bis 30 % beträgt.

5. Verfahren nach Anspruch 4, dadurch gekennzeichnet, daß der Feststoffgehalt der Suspension 15 bis 20 % beträgt.

6. Verfahren nach einem der Ansprüche 1 bis 5, dadurch gekennzeichnet, daß die Inertisierung in drei aufeinanderfolgenden Reaktionsräumen erfolgt.

7. Verfahren nach einem der Ansprüche 1 bis 6, dadurch gekennzeichnet, daß die Aufenthaltsdauer der Suspension in den Reaktionsräumen in Abhängigkeit von Art und Menge der organischen Stoffe insgesamt etwa 40 bis 80 Tage beträgt.

8. Verfahren nach einem der Ansprüche 1 bis 7, dadurch gekennzeichnet, daß die Suspension vor der Einspeisung in den jeweils ersten Reaktionsraum einer Hydrolyse und Versäuerung unterzogen wird.

9. Verfahren nach einem der Ansprüche 1 bis 8, dadurch gekennzeichnet, daß die Suspension vor und/oder parallel zu der Inertisierung einer Behandlung unter aeroben Bedingungen derart unterzogen wird, daß die unter anaeroben Bedingungen schwer abbaubaren Stoffe, wie z.B. Lignin, durch aerobe Mikroorganismen so aufgeschlossen werden, daß sie dem anaeroben Abbauprozeß zugänglich werden.

10. Verfahren nach Anspruch 9, dadurch gekennzeichnet, daß die Behandlung unter aeroben Bedingungen vor der Hydrolyse und unter Einsatz von auf die Mineralisierung der schwer abbaubaren Stoffe spezialisierten Mikroorganismen, z.B. Pilzen, vorzugsweise Weißfäulepilzen, erfolgt und durch Einstellung des pH-Wertes so geführt wird, daß die Mikroorganismen das Optimum ihrer Enzymaktivität aufweisen und gleichzeitig die Mineralisierung der leicht angreifbaren organischen Substanzen durch Bakterien weitgehend eingeschränkt ist.

11. Verfahren nach Anspruch 10, dadurch gekennzeichnet, daß solche Stämme von Mikroorganismen eingesetzt werden, die einen hohen Quotienten zwischen ligninolytischer und cellulolytischer Aktivität aufweisen.

12. Verfahren nach einem der Ansprüche 9 bis 11, dadurch gekennzeichnet, daß die Behandlung bei einem pH-Wert von etwa 4 bis 5 erfolgt.

13. Verfahren nach Anspruch 12, dadurch gekennzeichnet, daß die Behandlung bei einem pH-Wert von 4,2 bis 4,6 erfolgt.

14. Verfahren nach einem der Ansprüche 9 bis 13, dadurch gekennzeichnet, daß zur Einstellung des pH-Wertes ein organischer Puffer, vorzugsweise Succinat und Phthalat, zudosiert oder Kohlendioxid eingeleitet wird.

15. Verfahren nach Anspruch 9, dadurch gekennzeichnet, daß die Behandlung unter aeroben Bedingungen parallel zu der anaeroben Behandlung erfolgt.

16. Verfahren nach Anspruch 15, dadurch gekennzeichnet, daß die Behandlung nach dem ersten, vorzugsweise parallel zu dem jeweils zweiten Reaktionsraum erfolgt.

17. Verfahren nach einem der Ansprüche 9 bis 16, dadurch gekennzeichnet, daß die aerobe Behandlung in einem Airliftschlaufenreaktor erfolgt.

18. Verfahren nach einem der Ansprüche 2 bis 17, dadurch gekennzeichnet, daß das zur Regulierung des Feststoffgehalts entnommene, organisch belastete Überschußwasser zur Inertisierung einer gesonderten Behandlung unter anaeroben Bedingungen unterzogen wird.

19. Verfahren nach Anspruch 18, dadurch gekennzeichnet, daß die Behandlung in einem Hochleistungs-Festbett-Methanreaktor erfolgt.

20. Verfahren nach einem der Ansprüche 1 bis 19, dadurch gekennzeichnet, daß das Überschußwasser aus der Regulierung des Feststoffgehalts und gegebenenfalls einer abschließenden Reststoffentwässerung als Prozeßwasser im Kreislauf geführt wird.

21. Verfahren nach einem der Ansprüche 1 bis 20, dadurch gekennzeichnet, daß zur Vergleichmäßigung des Gehalts der Suspension an organischer Substanz und Stabilisierung des anaeroben Abbauprozesses organisches Material, z.B. Melasse, zugegeben wird.
